# EUROPÄISCHE PATENTANMELDUNG

(11) **EP 4 079 332 A1**
(43) Veröffentlichungstag der Anmeldung: **26.10.2022**
(21) Anmeldenummer: 22168312.1
(22) Anmeldetag: 14.04.2022
(51) Int. Cl.: A61L 2/00, A61L 2/18, A61L 9/012, A61L 9/14, B05B 11/00

(54) **PORTABLER FLÜSSIGKEITSSPENDER**

(30) Priorität: 19.04.2021 DE 102021109837
(71) Anmelder: Wrobel, Alexander, 44329 Dortmund (DE)
(72) Erfinder: Wrobel, Alexander, 44329 Dortmund (DE)
(74) Vertreter: Kalkoff & Partner Patentanwälte mbB

(57) **Zusammenfassung**

Die Erfindung betrifft einen portablen Flüssigkeitsspender, insbesondere Desinfektionsmittelspender, mit einem zur Aufnahme eines Flüssigkeitsbehälters ausgebildeten Spendergrundkörper mit einer Flüssigkeitsaustrittsöffnung und einer Befüllöffnung und einem die Befüllöffnung verschließenden Deckelelement. Um einen portablen Flüssigkeitsspender bereitzustellen, welcher eine zuverlässige Blockierung der Abgabe der Flüssigkeit mit einem einfachen Sperrmechanismus ermöglicht, ist vorgesehen, dass das Deckelelement derart über einen Kopplungsabschnitt mit dem Spendergrundkörper verbunden ist, dass das Deckelelement mit dem Spendergrundkörper zwischen
- einer das Deckelelement gegenüber dem Spendergrundkörper freigebenden Entnahmestellung,
- einer eine axiale Verlagerung des Deckelelements gegenüber dem Spendergrundkörper blockierenden Verriegelungsstellung und
- einer das Deckelelement zur axialen Verlagerung bis in eine Endlage freigebenden Betätigungsstellung

verstellbar ist.

## Beschreibung

Die Erfindung betrifft einen portablen Flüssigkeitsspender, insbesondere Desinfektionsmittelspender, mit
- einem zur Aufnahme eines Flüssigkeitsbehälters ausgebildeten Spendergrundkörper mit einer Flüssigkeitsaustrittsöffnung und einer Befüllöffnung und
- einem die Befüllöffnung verschließenden Deckelelement.

Portable Desinfektionsmittelspender der eingangs genannten Art sind in vielfältigen Ausgestaltungen aus dem Stand der Technik bekannt und werden bspw. dafür verwendet, um an unterschiedlichen Orten zu unterschiedlichen Zeitpunkten die Hände zu Desinfizieren. Im Hinblick auf die COVID-19 Pandemie hat die Bedeutung von portablen Desinfektionsmittelspendern weiter zugenommen.

Zum portablen Transport weisen Desinfektionsmittelspender üblicherweise eine transportierbare Gesamtgröße auf, so dass sie bspw. in einer Handtasche oder an einem Rucksack transportiert werden können. Bekannte Ausführungsformen portabler Desinfektionsmittelspender weisen einen Behälter zur Aufnahme des Desinfektionsmittels, z.B. Gel oder Flüssigkeiten und wahlweise ein Gehäuse mit einem Deckel auf. Der Deckel weist üblicherweise einen Pumpmechanismus auf, der bei Betätigung das Desinfektionsmittel aus dem Behälter abgibt. Einige Desinfektionsmittelspender weisen weiterhin einen separaten Sperrmechanismus zur Blockierung des Pumpmechanismus auf, damit aus dem Desinfektionsmittelspender während des Transports nicht unbeabsichtigt Desinfektionsmittel austritt. Bekannte separate Sperrmechanismen sind in ihrer Konstruktion sehr aufwendig und weisen den Nachteil auf, dass sie aus mehreren Komponenten bestehen. Zudem bieten sie nur einen unzureichenden Schutz vor einem unbeabsichtigten Austritt des Desinfektionsmittels.

Hiervon ausgehend liegt der Erfindung die Aufgabe zugrunde, einen portablen Flüssigkeitsspender, insbesondere Desinfektionsmittelspender, bereitzustellen, welcher eine zuverlässige Blockierung der Abgabe der Flüssigkeit, insbesondere Desinfektionsmittel, mit einem einfachen Sperrmechanismus ermöglicht.

Die Erfindung löst die Aufgabe durch einen portablen Flüssigkeitsspender mit den Merkmalen des Anspruchs 1. Vorteilhafte Weiterbildungen der Erfindung sind in den abhängigen Ansprüchen angegeben.

Kennzeichnend für den erfindungsgemäßen portablen Flüssigkeitsspender ist, dass das Deckelelement derart über einen Kopplungsabschnitt mit dem Spendergrundkörper verbunden ist, dass das Deckelelement mit dem Spendergrundkörper zwischen
- einer das Deckelelement gegenüber dem Spendergrundkörper freigebenden Entnahmestellung,
- einer eine axiale Verlagerung des Deckelelements gegenüber dem Spendergrundkörper blockierenden Verriegelungsstellung und
- einer das Deckelelement zur axialen Verlagerung bis in eine Endlage freigebenden Betätigungsstellung
verstellbar ist.

Der hülsenförmige Spendergrundkörper weist eine Innenfläche und eine Außenfläche auf, wobei der von der Innenfläche abgegrenzte Innenraum des Spendergrundkörpers zur Aufnahme des Flüssigkeitsbehälters, insbesondere Desinfektionsmittelbehälters, ausgebildet ist, welcher über die Befüllöffnung des Spendergrundkörpers in dem Innenraum angeordnet ist und aus diesem entnommen werden kann. Der Durchmesser der Befüllöffnung, welche eine runde oder eckige Form aufweist, ist so definiert, dass der Flüssigkeitsbehälter komfortabel im Spendergrundkörper positioniert werden kann. Bei dem Flüssigkeitsbehälter handelt es sich um einen Behälterkörper, welcher mit einer Flüssigkeit, insbesondere Desinfektionsmittel, befüllt ist. Dieser kann, bspw. mit einer Kappe oder dergleichen separat verschlossen werden, wobei die Kappe vor der Anordnung von dem Behälterkörper entfernt wird.

Zum Verschließen des Spendergrundkörpers wird das Deckelelement im Bereich der Befüllöffnung mit dem Spendergrundkörper verbunden, so dass das Deckelement die Befüllöffnung verschließt. Das Deckelelement ist dabei über den Kopplungsabschnitt gegenüber dem Spendergrundkörper zwischen drei verschiedenen Stellungen verstellbar.

In der Entnahmestellung befinden sich das Deckelelement und der Spendergrundkörper derart über den Kopplungsabschnitt im Eingriff, dass das Deckelelement von dem Spendergrundkörper getrennt werden kann und so die Befüllöffnung freigibt.

Aus der Entnahmestellung ist das Deckelelement ferner in eine Verriegelungsstellung verstellbar, in der eine axiale Verlagerung des Deckelelements blockiert ist. D.h., das Deckelelement kann nicht axial in Richtung auf die Befüllöffnung oder in die entgegengesetzte Richtung bewegt werden.

Zur axialen Verlagerung des Deckelements in Richtung auf das der Befüllöffnung gegenüberliegende Ende ist der Kopplungsabschnitt ferner derart ausgebildet, dass das Deckelelement über den Kopplungsabschnitt in die Betätigungsstellung verlagerbar ist. Ausgehend von der Betätigungsstellung ist das Deckelelement axial gegenüber dem Spendergrundkörper in die Endlage verstellbar, wobei das Deckelelement dabei derart auf einen am Spendergrundkörper angeordneten Flüssigkeitsbehälter wirkt, dass Flüssigkeit aus dem Flüssigkeitsbehälter über die Flüssigkeitsaustrittsöffnung aus dem Flüssigkeitsspender heraus befördert wird.

Im Ergebnis ergibt sich ein portabler Flüssigkeitsspender, insbesondere Desinfektionsmittelspender, mit einem Spendergrundkörper, einem Deckelelement und einem Kopplungsabschnitt mit drei Stellungen. Die Entnahmestellung ermöglicht die einfache Montage, bzw. Demontage von Spendergrundkörper und Deckelelement sowie eine einfache Entnahme und Positionierung des Flüssigkeitsbehälters. Die Verriegelungsstellung verhindert ein ungeplantes Abgeben der Flüssigkeit über die Flüssigkeitsaustrittsöffnung. Um die funktionsgemäße Abgabe der Flüssigkeit über die Flüssigkeitsaustrittsöffnung zu gewährleisten, ermöglicht die Betätigungsstellung die axiale Verlagerung des Deckelements.

Die Ausgestaltung des Kopplungsabschnitts ist dabei grundsätzlich frei wählbar. So kann dieser bspw. durch eine an dem einen von Spendergrundkörper und Deckelelement angeordnete Nut gebildet sein, die mit einem Nutkörper an dem anderen von Spendergrundkörper und Deckelelement zusammenwirkt. Nut und Nutkörper bilden dann gemeinsam den Kopplungsabschnitt. Über den Verlauf der Nut werden die verschiedenen Stellungen des Deckelelements festgelegt.

Nach einer besonders vorteilhaften Ausgestaltung der Erfindung ist jedoch vorgesehen, dass der Kopplungsabschnitt ein an dem einen von Spendergrundkörper und Deckelelement angeordnetes Innengewinde und ein an dem anderen von Spendergrundkörper und Deckelelement angeordnetes, mit dem Innengewinde in Eingriff bringbares Außengewinde aufweist. Das Innengewinde und das Außengewinde befinden sich in der Entnahmestellung derart im Eingriff, dass das Deckelelement vom Spendergrundkörper lösbar ist. In der Verriegelungsstellung sind das Innengewinde und das Außengewinde durch eine Verdrehung des Deckelelements relativ zum Spendergrundkörper derart im Eingriff, dass die axiale Verlagerung des Deckelelements gegenüber dem Spendergrundkörper blockiert ist. In der Betätigungsstellung liegen das Innengewinde und das Außengewinde wiederum derart aneinander an, dass das Deckelelement nicht vom Spendergrundkörper gelöst werden kann. Ein Wechsel von der Entnahmestellung über die Verriegelungsstellung zur Betätigungsstellung erfolgt durch eine Rotationsbewegung des Deckelelements oder des Spendergrundkörpers gegenüber dem Spendergrundkörper oder dem Deckelement. Das Innengewinde und das Außengewinde sind für einen Wechsel zwischen den drei Stellungen vorzugsweise offen ausgebildet. Das bedeutet, dass das Innengewinde oder das Außengewinde entlang der Längsachse des Spendergrundkörpers mit dem Außengewinde oder dem Innengewinde in beide Richtungen für den Eingriff offen ist. Der Vorteil eines Gewindes als Kopplungsabschnitt liegt in der kostengünstigen Herstellung und der einfachen Handhabung. Der Wechsel zwischen den drei Stellungen ist schnell möglich und im portablen Einsatz vorteilhaft, da bei Bedarf der Wechsel in die Betätigungsstellung einfach realisiert werden kann.

Nach einer Weiterbildung der Erfindung weist der Flüssigkeitsspender einen am Deckelelement oder Spendergrundkörper angeordneten, an dem Spendergrundkörper oder dem Deckelelement in der Endlage anliegenden Absatz auf.

Dieser Absatz ist bevorzugt an der Außenfläche des Spendergrundkörpers oder an der Außenfläche des Deckelelements, besonders bevorzugt an der Innenfläche des Spendergrundkörpers oder der Innenfläche des Deckelelements ausgebildet.

Durch die Ausbildung eines Absatzes kann in zuverlässiger Weise die Endlage des Deckelelements gegenüber dem Spendergrundkörper in einfacher Weise festgelegt werden. Auf separate Vorrichtungen zur Festlegung der Endlage kann somit verzichtet werden.

Nach einer vorteilhaften Weiterbildung der Erfindung ist vorgesehen, dass neben dem Spendergrundkörper auch das Deckelelement zur abschnittsweisen Aufnahme des Flüssigkeitsbehälters, insbesondere Desinfektionsmittelbehälters, ausgebildet ist, wobei das Deckelement dann bereichsweise koaxial zum Flüssigkeitsbehälter angeordnet ist. Durch diese vorteilhafte Weiterbildung kann die Länge des Spendergrundkörpers reduziert werden. Hierzu weist das Deckelelement bevorzugt mindestens eine Flüssigkeitsbehälterführungsfläche auf. Diese Führungsfläche ist bevorzugt eine Innenfläche des Deckelelements die besonders bevorzugt senkrecht zu einer Deckelöffnung des Deckelelements angeordnet ist.

Die Anordnung der Flüssigkeitsaustrittsöffnung ist grundsätzlich frei wählbar. So kann diese bspw. an einer Seite des Spendergrundkörpers angeordnet sein. Gemäß einer vorteilhaften Ausgestaltung der Erfindung ist die Flüssigkeitsaustrittsöffnung jedoch im Bereich eines der Befüllöffnung gegenüberliegenden Abschnitts des Spendergrundkörpers, besonders bevorzugt koaxial zur Längsachse des Spendergrundkörpers angeordnet. Die Längsachse des Spendergrundkörpers verläuft vorzugsweise zentral durch die Befüllöffnung und kann ebenso lotrecht zu dieser stehen. Eine entsprechende Anordnung der Flüssigkeitsaustrittsöffnung ermöglicht in besonders zuverlässiger Weise eine gerichtete Abgabe der Flüssigkeit, insbesondere Desinfektionsmittel, wodurch einer Fehlplatzierung wirksam vorgebeugt wird.

Nach einer Weiterbildung der Erfindung ist die Flüssigkeitsaustrittsöffnung an die Form des Flüssigkeitsbehälters angepasst. Beispielsweise ist die Flüssigkeitsaustrittsöffnung eine Bohrung, die besonders bevorzugt mit einer Öffnung am Flüssigkeitsbehälter deckungsgleich ist. Die Flüssigkeitsaustrittsöffnung kann z.B. eine Form aufweisen, in der ein Abschnitt des Flüssigkeitsbehälters positioniert ist. Eine angepasste Form gemäß dieser Weiterbildung der Erfindung gewährleistet ferner eine erhebungsfreie Fläche des Spendergrundkörpers im Bereich der Flüssigkeitsaustrittsöffnung, wodurch der Flüssigkeitsspender eine besonders gute Haptik aufweist.

Nach einer besonders vorteilhaften Ausgestaltung der Erfindung kann der Flüssigkeitsspender eine Verschlusseinheit mit einem Verschlusselement aufweisen, das bevorzugt zwischen einer die Flüssigkeitsaustrittsöffnung freigebenden Öffnungsstellung und einer die Flüssigkeitsaustrittsöffnung verschließenden Schließstellung verstellbar ist. Die Verschlusseinheit ist beispielsweise eine Lochplatte mit einer Scheibe, die bevorzugt als Verschlusselement die Flüssigkeitsaustrittsöffnung freigeben oder verschließen kann. Das Verschlusselement kann z.B. zwischen der Öffnungsstellung und der Schließstellung verschiebbar oder verschwenkbar sein. Ebenfalls ist das Verschlusselement beispielsweise manuell bedienbar. Für die Bedienung des Verschlusselementes kann z.B. ein Schieber oder bevorzugt ein Rad am Spendergrundkörper angeordnet sein. Ein separater Verschluss der Flüssigkeitsaustrittsöffnung verhindert in besonders zuverlässiger Weise eine unplanmäßige Abgabe von Flüssigkeiten zusätzlich zur Sperrstellung des Kopplungsabschnitts. Die Verschlusseinheit bietet somit einen weiteren Schutz für den Anwender vor einem ungewollten Austritt von Flüssigkeit, insbesondere Desinfektionsmittel.

Deckelelement und Spendergrundkörper können aus beliebigen Werkstoffen gebildet sein, die durch ihr geringes Eigengewicht die portable Benutzung des Flüssigkeitsspenders begünstigen. Solche Materialien sind beispielsweise Kunststoffe oder Verbundwerkstoffe mit Glasfasern. Gemäß einer besonders bevorzugten Weiterbildung sind das Deckelelement und der Spendergrundkörper aus einem Aluminiumwerkstoff gebildet. Der Einsatz von Aluminiumwerkstoffen bietet die Möglichkeit, einen als Desinfektionsmittelspender ausgebildeten Flüssigkeitsspender bei der Benutzung mit zu desinfizieren. Zudem lässt sich der Flüssigkeitsspender besonders einfach und kostengünstig herstellen.

Bevorzugt kann der Spendergrundkörper und/oder das Deckelelement ein Führungselement aufweisen. Bei den Führungselementen handelt es sich beispielsweise um Nuten an Spendergrundkörper oder Deckelelement, bevorzugt kombiniert mit Streben an Spendergrundkörper oder Deckelelement. Das Führungselement kann auch ein zur Längsachse des Spendergrundkörpers parallel verlaufender Absatz an der Innenfläche des Spendergrundkörpers sein. Mit einem Führungselement kann die axiale Verlagerung des Deckelelements besonders vorteilhaft geführt werden, wodurch einem Verkanten besonders wirksam vorgebeugt wird.

Nach einer bevorzugten Ausführungsform der Erfindung ist der der Befüllöffnung gegenüberliegende Abschnitt des Spendergrundkörpers kugelkalottenförmig ausgebildet. Eine Alternative kann beispielsweise eine eckige Gestaltung dieses Abschnitts sein. Der Vorteil in einer kugelkalottenförmigen Ausführungsform liegt in einer besonders guten Haptik für den Anwender.

Nach einer weiteren Ausgestaltung der Erfindung weist der Spendergrundkörper einen konischen Innenabschnitt auf. Durch eine entsprechende Ausgestaltung kann der Spendergrundkörper über den Innenabschnitt zentrierend auf den Flüssigkeitsbehälter wirken, so dass dieser besonders exakt gegenüber der Flüssigkeitsaustrittsöffnung positioniert ist.

Nach einer vorteilhaften Weiterbildung der Erfindung weist der Spendergrundkörper bevorzugt einen von einer Innenfläche vorstehenden Vorsprung auf, der besonders bevorzugt als Anlagefläche für den Flüssigkeitsbehälter ausgebildet ist. Der Vorsprung gewährleistet in besonders zuverlässiger Weise eine exakte Ausrichtung des Flüssigkeitsbehälters in Längsachsenrichtung des Spendergrundkörpers. Der Vorsprung ist eine Anlagefläche für die Pumpbewegung des Flüssigkeitsbehälters, wenn dieser entsprechend ausgebildet ist. Bspw. ist der Flüssigkeitsbehälter ein für Nasensprays oder Augentropfen bekannter Behälter mit integrierter Pumpfunktion.

Das Deckelelement ist nach einer Weiterbildung der Erfindung in Richtung auf die Befüllöffnung vorgespannt. Besonders bevorzugt weist der Flüssigkeitsspender hierzu ein einenends an dem Deckelelement und anderenends an dem Spendergrundkörper anliegendes Federelement auf. Diese Ausgestaltung gewährleistet unabhängig von der Ausführung des Flüssigkeitsbehälters eine zuverlässige Rückverlagerung des Deckelelements aus der Endlage in die Betätigungsstellung. Der Anwender kann allein durch die von ihm auf das Deckelelement wirkende Druckkraft den Flüssigkeitsspender bedienen, unabhängig von der Ausgestaltung des Flüssigkeitsbehälters, bspw. wenn dieser selbst eine Pumpbewegung vorsieht.

In einer weiteren Ausgestaltung der Erfindung weist der Spendergrundkörper und/oder das Deckelelement eine Öse für ein Halteband auf. Die Öse ist bevorzugt an der Außenfläche des Spendergrundkörpers oder der Außenfläche des Deckelelements angeordnet. Bevorzugt ist die Öse über ein Verbindungselement mit dem Spendergrundkörper oder dem Deckelelement verbunden. Das Verbindungselement ist z.B. ein Stift. Besonders bevorzugt ist die Öse mit dem Deckelelement oder dem Spendergrundkörper einstückig ausgebildet. Die Öse ermöglicht es, z.B. ein Stoffband, eine Kette, ein Lederband oder dergleichen an dem Flüssigkeitsspender zu befestigen. Somit kann der portable Flüssigkeitsspender, insbesondere Desinfektionsmittelspender, beispielsweise an einer Tasche, einem Rucksack oder besonders bevorzugt an einer Gürtelschlaufe befestigt und damit komfortabel mitgeführt werden.

Ein Ausführungsbeispiel der Erfindung wird nachstehend mit Bezug auf die Zeichnungen erläutert. In den Zeichnungen zeigen:
Figur 1 eine perspektivische Ansicht eines Flüssigkeitsspenders;
Figur 2 eine Seitenansicht des Flüssigkeitsspenders von Figur 1;
Figur 3 eine Ansicht eines Längsschnitts durch den Flüssigkeitsspender entlang der Linie A-A von Figur 2;
Figur 4 eine Explosionszeichnung des Flüssigkeitsspenders von Figur 1;

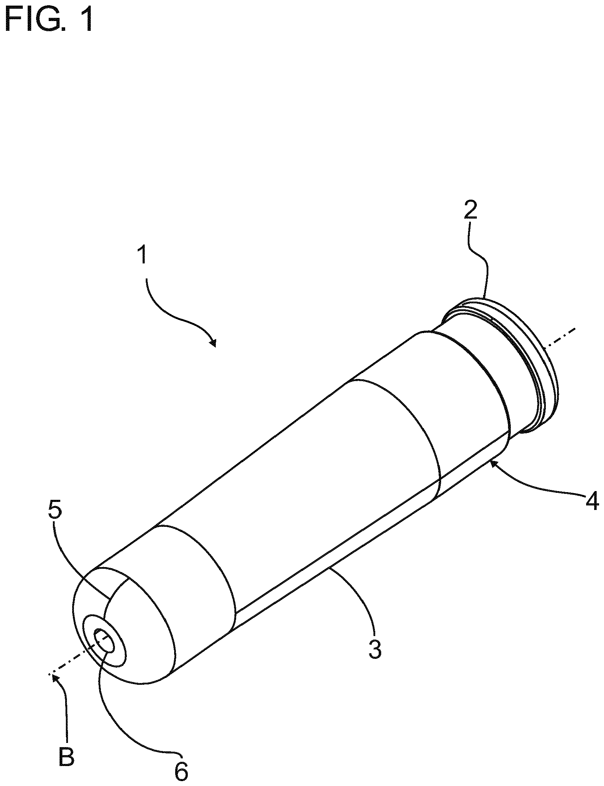

Figur 1 zeigt in perspektivischer Ansicht eine Ausführungsform eines Flüssigkeitsspenders 1. Der Flüssigkeitsspender 1 weist ein Deckelelement 2 und einen Spendergrundkörper 3 auf, die aus einem Aluminiumwerkstoff gebildet sind. Über einen innenliegenden Kopplungsabschnitt 4 ist das Deckelelement 2 gegenüber dem Spendergrundkörper 3 zwischen drei Stellungen verstellbar.

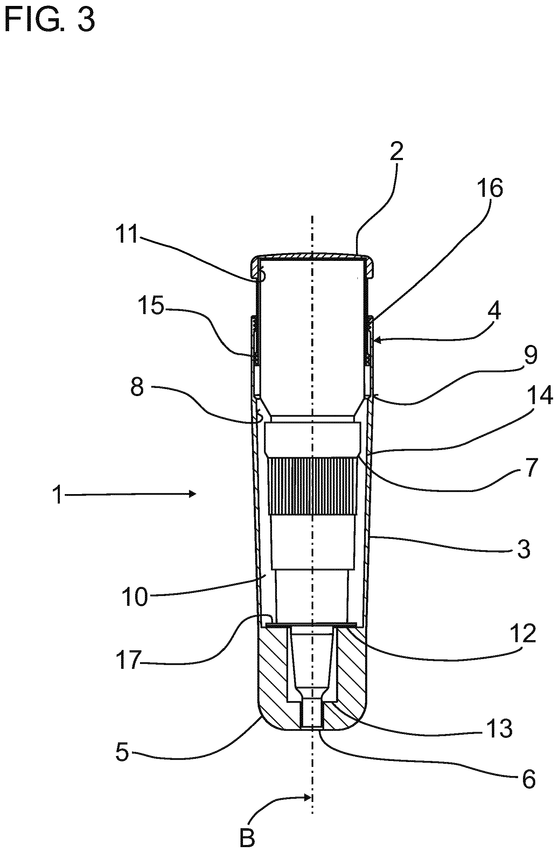

Der hülsenförmige Spendergrundkörper 3 weist an der dem Deckelelement 2 gegenüberliegenden Seite einen kugelkalottenförmigen Abschnitt 5 und koaxial zu einer Längsachse B des Spendergrundkörpers 3 eine Flüssigkeitsaustrittsöffnung 6 auf. Die Flüssigkeitsaustrittsöffnung 6 ist eine Bohrung, die an die Form eines Flüssigkeitsbehälters 7 im Bereich seiner Austrittsöffnung (in Fig. 3 schematisch dargestellt) angepasst ist.

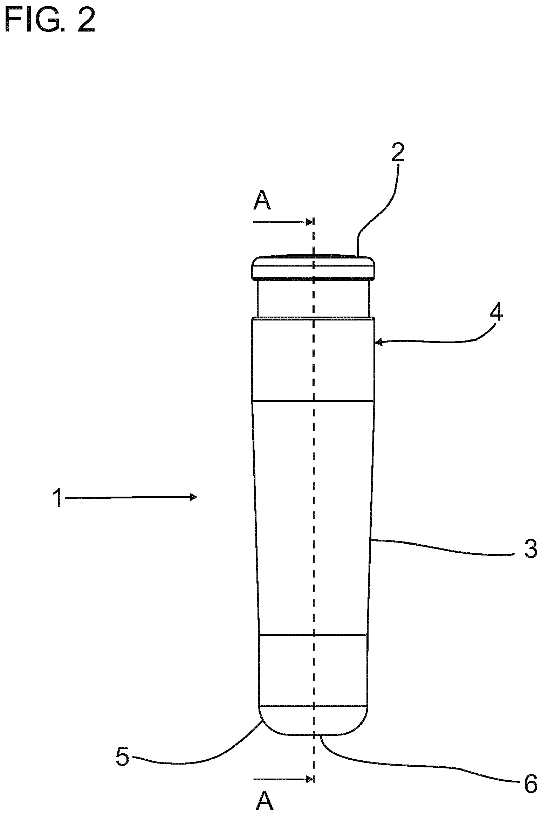

Der Spendergrundkörper 3 weist eine Innenfläche 8 und eine Außenfläche 9 auf. Ein von der Innenfläche 8 des Spendergrundkörpers 3 abgegrenzter Innenraum 10 ist für die Aufnahme des Flüssigkeitsbehälters 7 (nicht geschnitten dargestellt) ausgebildet. Der Flüssigkeitsbehälter 7 weist in der dargestellten Ausführungsform die Form an sich bekannter Behälter auf, wie sie bspw. für Nasensprays oder Augentropfen bekannt sind. Das Deckelement 2 und der Spendergrundkörper 3 sind zur teilweisen Aufnahme des Flüssigkeitsbehälters 7 ausgebildet. Das Deckelelement 2 weist eine Innenfläche in Form einer Führungsfläche 11 auf. Im Innenraum 10 des Spendergrundkörpers 3 sind ein Absatz 12 und ein Vorsprung 13 angeordnet. Weiterhin weist der Spendergrundkörper 3 einen konischen Innenabschnitt 14 auf. Dieser ermöglicht mit der an den Flüssigkeitsbehälter 7 angepassten Flüssigkeitsaustrittsöffnung 6 und dem Vorsprung 13 eine Zentrierung des Flüssigkeitsbehälters 7 im Innenraum 10 des Spendergrundkörpers 3. (vgl. Fig. 2)

Der Kopplungsabschnitt 4 ist durch ein am Deckelelement 2 angeordnetes Außengewinde 15 und ein am Spendergrundkörper 3 angeordnetes Innengewinde 16 gebildet.

Das Deckelelement 2 ist über das Außengewinde 15 mit dem Innengewinde 16 des Spendergrundkörpers 3 verbunden. Durch Drehbewegungen des Deckelelements 2 gegenüber dem Spendergrundkörper 3 kann das Deckelelement 2 zwischen einer Entnahmestellung, einer Verriegelungsstellung und einer Betätigungsstellung verstellt werden.

Hierfür sind das Außengewinde 15 und das Innengewinde 16 offen gestaltet, so dass gewährleistet ist, dass z.B. das Außengewinde 15 von beiden Richtungen aus um die Längsachse A des Spendergrundkörpers 3 in das Innengewinde 16 einschraubbar ist.

In der Entnahmestellung befinden sich Außengewinde 15 und Innengewinde 16 derart im Eingriff, dass das Deckelelement 2 vom Spendergrundkörper 3 lösbar ist. Eine Drehbewegung des Deckel elements 2 gegenüber dem Spendergrundkörper 3 um die Längsachse B des Spendergrundkörpers 3, wobei Innengewinde 16 und Außengewinde 15 in einem zueinander verschraubten Eingriff befindlich sind, führt zum Wechsel in die Verriegelungsstellung. In der Verriegelungsstellung ist eine axiale Verlagerung des Deckelelements 2 gegenüber dem Spendergrundkörper 3 blockiert. Somit kann das Deckelement 2 nicht axial in Richtung auf die Flüssigkeitsaustrittsöffnung 6 oder die entgegengesetzte Richtung bewegt werden.

Der Wechsel in die Betätigungsstellung erfolgt über eine weitere Drehbewegung des Deckelelements 2 gegenüber dem Spendergrundkörper 3 um die Längsachse B des Spendergrundkörpers 3, bis das Außengewinde 15 und das Innengewinde 16 nicht mehr in einem zueinander verschraubten Eingriff befindlich sind. In der Betätigungsstellung ist das Deckelelement 2 axial gegenüber dem Spendergrundkörper 3 in Richtung auf die Endlage verstellbar. Bei einer Betätigung des Deckelelements 2 bewegt dieses bei der axialen Verlagerung den Flüssigkeitsbehälter 7 axial in Richtung der Flüssigkeitsaustrittsöffnung 6. Der Flüssigkeitsbehälter 7 liegt in der Betätigungsstellung des Deckelelements 2 mit einem überstehenden Abschnitt 17 auf dem Absatz 12 des Spendergrundkörpers 3 auf. Eine axiale Verlagerung des Deckelelements 2 in Richtung auf die Endlage wird auf den Flüssigkeitsbehälter 7 übertragen, wodurch dessen interner Pumpmechanismus ausgelöst wird und Flüssigkeit durch die Flüssigkeitsaustrittsöffnung 6 abgegeben wird.

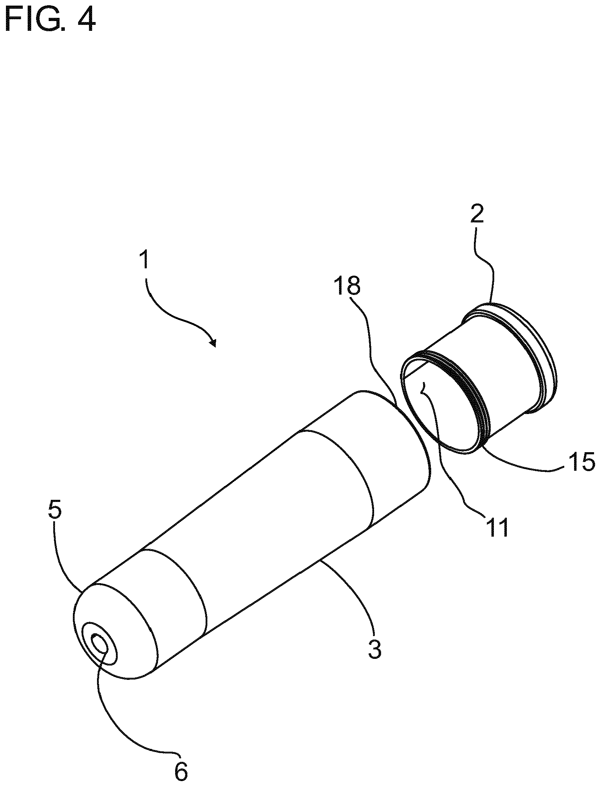

In der in Figur 4 dargestellten Explosionszeichnung des Flüssigkeitsspenders 1 sind das Deckelelement 2 und der Spendergrundkörper 3 in der Entnahmestellung voneinander gelöst.

Das Deckelelement 2 gibt eine Befüllöffnung 18 des Spendergrundkörpers 3 frei. Die Befüllöffnung 18 weist einen Durchmesser auf, der eine komfortable Positionierung des Flüssigkeitsbehälters 7 im Innenraum 10 des Spendergrundkörpers 3 ermöglicht.

### Bezugszeichenliste

- 1: Flüssigkeitsspender
- 2: Deckelelement
- 3: Spendergrundkörper
- 4: Kopplungsabschnitt
- 5: kugelkalottenförmiger Abschnitt des Spendergrundkörpers
- 6: Flüssigkeitsaustrittsöffnung
- 7: Flüssigkeitsbehälter
- 8: Innenfläche des Spendergrundkörpers
- 9: Außenfläche des Spendergrundkörpers
- 10: Innenraum des Spendergrundkörpers
- 11: Führungsfläche des Deckelelements
- 12: Absatz im Innenraum des Spendergrundkörpers
- 13: Vorsprung im Innenraum des Spendergrundkörpers
- 14: konischer Abschnitt im Innenraum des Spendergrundkörpers
- 15: Außengewinde
- 16: Innengewinde
- 17: Abschnitt des Flüssigkeitsbehälters
- 18: Befüllöffnung
- B: Längsachse des Spendergrundkörpers

## Patentansprüche

1. Portabler Flüssigkeitsspender (1), insbesondere Desinfektionsmittelspender, mit
- einem zur Aufnahme eines Flüssigkeitsbehälters (7) ausgebildeten Spendergrundkörper (3) mit einer Flüssigkeitsaustrittsöffnung (6) und einer Befüllöffnung (18) und
- einem die Befüllöffnung (18) verschließenden Deckelelement (2),
**dadurch gekennzeichnet, dass** das Deckelelement (2) derart über einen Kopplungsabschnitt (4) mit dem Spendergrundkörper (3) verbunden ist, dass das Deckelelement (2) mit dem Spendergrundkörper (3) zwischen
- einer das Deckelelement (2) gegenüber dem Spendergrundkörper (3) freigebenden Entnahmestellung,
- einer eine axiale Verlagerung des Deckelelements (2) gegenüber dem Spendergrundkörper (3) blockierenden Verriegelungsstellung und
- einer das Deckelelement (2) zur axialen Verlagerung bis in eine Endlage freigebenden Betätigungsstellung
verstellbar ist.

2. Flüssigkeitsspender nach Anspruch 1, **dadurch gekennzeichnet, dass** der Kopplungsabschnitt (4) ein an dem einen von Spendergrundkörper (3) und Deckelelement (2) angeordnetes Innengewinde (16) und ein an dem anderen von Spendergrundkörper (3) und Deckelelement (2) angeordnetes, mit dem Innengewinde (16) in Eingriff bringbares Außengewinde (15) aufweist.

3. Flüssigkeitsspender nach Anspruch 1 oder 2, **gekennzeichnet durch** einen am Deckelelement (2) oder Spendergrundkörper (3) angeordneten, an dem Spendergrundkörper (3) oder dem Deckelelement (2) in der Endlage anliegenden Absatz (12).

4. Flüssigkeitsspender nach einem oder mehrerer der vorherigen Ansprüche, **dadurch gekennzeichnet, dass** das Deckelelement (2) eine mit dem Flüssigkeitsbehälter (7) in Eingriff bringbare Führungsfläche (11) aufweist.

5. Flüssigkeitsspender nach einem oder mehrerer der vorherigen Ansprüche, **dadurch gekennzeichnet, dass** die Flüssigkeitsaustrittsöffnung (6) im Bereich eines der Befüllöffnung (18) gegenüberliegenden Abschnitts (5) des Spendergrundkörpers (3) angeordnet ist.

6. Flüssigkeitsspender nach einem oder mehrerer der vorherigen Ansprüche, **dadurch gekennzeichnet, dass** die Flüssigkeitsaustrittsöffnung (6) koaxial zur Längsachse (B) des Spendergrundkörpers (3) angeordnet ist.

7. Flüssigkeitsspender nach einem oder mehrerer der vorherigen Ansprüche, **dadurch gekennzeichnet, dass** die Flüssigkeitsaustrittsöffnung (6) an die Form des Flüssigkeitsbehälters (7) angepasst ist.

8. Flüssigkeitsspender nach einem oder mehrerer der vorherigen Ansprüche **gekennzeichnet durch** eine Verschlusseinheit mit einem Verschlusselement, das zwischen einer die Flüssigkeitsaustrittsöffnung (6) freigebenden Öffnungsstellung und einer die Flüssigkeitsaustrittsöffnung (6) verschließenden Schließstellung verstellbar ist.

9. Flüssigkeitsspender nach einem oder mehrerer der vorherigen Ansprüche, **dadurch gekennzeichnet, dass** das Deckelelement (2) und der Spendergrundkörper (3) aus einem Aluminiumwerkstoff gebildet sind.

10. Flüssigkeitsspender nach einem oder mehrerer der vorherigen Ansprüche, **dadurch gekennzeichnet, dass** der Spendergrundkörper (3) und/oder das Deckelelement (2) ein Führungselement aufweisen.

11. Flüssigkeitsspender nach einem oder mehrerer der vorherigen Ansprüche, **dadurch gekennzeichnet, dass** der der Befüllöffnung (18) gegenüberliegende Abschnitt (5) des Spendergrundkörpers (3) kugelkalottenförmig ausgebildet ist.

12. Flüssigkeitsspender nach einem oder mehrerer der vorherigen Ansprüche, **dadurch gekennzeichnet, dass** der Spendergrundkörper (3) einen konischen Innenabschnitt (14) aufweist.

13. Flüssigkeitsspender nach einem oder mehrerer der vorherigen Ansprüche, **dadurch gekennzeichnet, dass** der Spendergrundkörper (3) einen von einer Innenfläche (8) vorstehenden Vorsprung (13) aufweist, der als Anlagefläche für den Flüssigkeitsbehälter (7) ausbildet ist.

14. Flüssigkeitsspender nach einem oder mehrerer der vorherigen Ansprüche, **dadurch gekennzeichnet, dass** das Deckelelement (2) in Richtung auf die Befüllöffnung (18) vorgespannt ist.

15. Flüssigkeitsspender nach einem oder mehrerer der vorherigen Ansprüche, **gekennzeichnet durch** ein einenends an dem Deckelelement (2) und anderenends an dem Spendergrundkörper (3) anliegendes Federelement.

16. Flüssigkeitsspender nach einem oder mehrerer der vorherigen Ansprüche, **dadurch gekennzeichnet, dass** der Spendergrundkörper (3) und/ oder das Deckelelement (2) eine Öse für ein Halteband aufweisen.
